# EUROPEAN PATENT APPLICATION

(11) **EP 4 749 347 A1**
(43) Date of publication of application: **27.05.2026**
(21) Application number: 25215500.7
(22) Date of filing: 13.11.2025
(51) Int. Cl.: G02B 27/00, A61B 3/13

(54) **OPTICAL GROUP FOR SURGICAL PROCEDURES AND SYSTEM FOR DEFOGGING A LENS**

(30) Priority: 20.11.2024 IT 202400026136
(71) Applicant: Meduri, Alessandro, 98167 Messina (IT); Russo, Alfio, Gabriele, 95024 Acireale (Catania) (IT)
(72) Inventor: RUSSO, Alfio Gabriele, 95024 ACIREALE (CATANIA) (IT)
(74) Representative: Girometti, Emiliano

(57) **Abstract**

System for defogging an optical group, characterized in that it comprises a fluid flow generation device for pressurizing a continuous fluid flow; a transport device connected to the generation device and for transporting the fluid flow outside the generation device; at least one fluid flow dispensing element connected to the transport device and having a nozzle for dispensing the fluid flow in laminar form on the surface of the lens.

## Description

The present invention relates to an optical group for surgical procedures and a system for defogging a lens of the device.

More in particular, the present invention refers to a system arranged to be mounted on an optical group and capable of defogging the lens of the device.

By optical group is meant a support structure comprising at least one lens. The optical group is used to improve the vision of operators who need it and/or as a means of protection in various work activities.

For example, the optical group is often used in surgery, as a means of protection and/or to improve the view of the area of interest.

In particular, the optical group is widely used in the ophthalmic field.

In fact, in ophthalmic surgery, the optical group associated with an ophthalmic microscope is used which allows to magnify and improve the operator's view on the affected area. For example, the pan-fundoscopic optical system, used to surgically operate on the posterior part of the eyeball, is widely used in this technical field. Such examinations typically concern the entire vitreous cavity including the vitreous body, the optic nerve head, the central (macula) and peripheral retina.

As is known, the optical group in specific conditions may fog up the lens reducing the visual field of the operator.

In the case of the pan-fundoscopic optical system, which as specified above is widely used in ophthalmic surgery to have an excellent view of the central and peripheral retina and the vitreous chamber of the eye, the fogging of a lens can compromise the correct surgical procedure.

In more detail, the pan-fundoscopic optical system comprises a microscope for ophthalmic surgery and a pan-fundoscopic system comprising two lenses of different size and shape.

In particular, a substantially flat upper lens and a lower lens with a more marked convex shape than the upper lens are provided; in general, the upper lens has larger sizes than the lower lens. The two lenses are aligned and spaced apart.

Typically, the two lenses are connected to each other by means of a support capable of defining a certain distance between the two lenses and favouring the vision of the central and peripheral retina and the vitreous chamber of the eye.

As mentioned, the pan-fundoscopic system is associated with an ophthalmic microscope and, in particular, the upper lens of the system is connected to the microscope.

Generally, the pan-fundoscopic system is used in a sterile environment (e.g. an operating room) and therefore requires to be fully sterilized; in particular the lenses of the pan-fundoscopic system may be subjected to a plurality of sterilization cycles.

Following the sterilization cycles, the upper and lower lenses degrade easily and quickly and, therefore, the lenses must be replaced frequently in order to guarantee an excellent visual quality of the retina corresponding to an excellent quality of the surgical operation.

This system is used in operating rooms during vitreo-retinal surgical procedures. In particular, the pan-fundoscopic optical system is used in proximity to the patient's eye, and specifically the lower lens of the pan-fundoscopic system is placed in proximity to the eye to be treated.

In general, the system is aligned vertically to the patient's eye to be corrected, when the patient is in the supine position.

However, this type of system has a limit in the effectiveness of the operation.

In fact, the lower lens being proximal to the patient periodically fogs up, limiting the surgeon's visual field during the operation.

In fact, the water vapour generated by the patient's body temperature and breathing adheres to the lower surface of the lower lens of the pan-fundoscopic optical system, thus limiting the surgeon's visual field.

In other words, the surface of the lens facing the patient's body tends to fog as a result of condensation formation caused by the patient's proximal presence.

As a result, the surgeon is forced to stop the surgical operation to defog the lens, reposition the system in the correct position by aligning it with the patient's eye, and restart the surgical operation.

Typically, the lens is defogged using gauze and fabrics that over time score the lens itself. As a result, the lens must be replaced frequently in order to ensure excellent visual quality of the retina.

In addition, the continuous interruption is therefore uncomfortable and complicates the entire surgical procedure.

This, moreover, causes a disturbance to the doctor that could negatively affect the final result of the surgical operation.

Consequently, the interruption of the operation and the subsequent repositioning of the system require total times to perform the entire surgical operation that are greater than the times required by the simple operation without interruptions.

Disadvantageously, the longer total times to perform the entire operation have an impact on the efficiency of the hospital system and, therefore, the hospital itself is inefficient in providing medical services.

In fact, the longer total times result in fewer procedures performed in a given operating room and, consequently, higher costs for each single surgical operation.

To solve this drawback, solutions are also proposed in which the lens is defogged by using a liquid solution and/or alcoholic solution.

In particular, this procedure is used and applied to the lower lens before the start of the surgical operation and, therefore, before the positioning and alignment of the pan-fundoscopic optical system with the patient's eye.

This solution may thus reduce the likelihood of water vapour forming on the lower surface of the lower lens and/or may extend the time from the instant the system is aligned with the patient's eye to the instant the aforementioned water vapour adheres.

Nevertheless, this solution is only feasible for short-term surgical operations.

Therefore, even this procedure is not without its drawbacks.

Note in particular that the use of liquid and/or alcoholic solutions may contaminate a sterile environment.

In addition, as specified above, the use of liquid and/or alcoholic solutions is only effective for a short duration. In fact, in the event that the procedure lasts for a prolonged time, the adhesion of water vapour may still occur even if the liquid and/or alcohol solution has previously been applied.

Therefore, the lens may fog up and require interruption of operation to defog the lens and subsequently reposition the system aligned to the patient's eye.

Consequently, even with these solutions, there could be a need for longer times for performing the surgical operation, a reduction in the number of procedures in a given operating room and, therefore, an increase in the cost of the single operation.

This implies a considerable drawback in terms of the efficiency of the hospital system and long times for performing the surgical operation.

In this context, the technical task underlying the present invention is to propose an optical group for surgical procedures and a system for defogging a lens that overcomes the drawbacks of the known technique mentioned above.

In more detail, an object of the present invention is to make available a system for effectively defogging a lens to keep the user's visual field always sharp.

Another main object of the present invention is to make available a system that can defog a lens for the entire duration of the procedure, regardless of the time necessary to conclude the procedure itself.

In other words, the object of the present invention is to make available a system that can minimize the need to interrupt the surgical operation, without requiring extended time to perform the operation and thus reducing the condition of operational discomfort.

Another object of the present invention is to make available a system that is positioned and aligned with the patient's eye only once, reducing the risk of inaccuracies in the positioning of the system that may affect the final result of the surgical operation.

It is still an object of the present invention to make available a system capable of reducing the total times of the operation, increasing the number of procedures for a given operating room and, consequently, reducing the costs of a single surgical operation.

It is still an object of the present invention to make available a system capable of reducing the need to perform sterilization cycles of an optical device and reducing lens degradation.

These and other objects are achieved by a system for defogging a lens comprising the technical features set forth in one or more of the appended claims.

In particular, the system subject-matter of the invention comprises a fluid flow generation device for pressurizing a continuous fluid flow; a transport device connected to the generation device and for transporting the fluid flow outside the generation device; at least one fluid flow dispensing element connected to the transport device and having a nozzle for dispensing the fluid flow in laminar form on the surface of the lens.

In a further aspect the invention relates to an optical group for surgical procedures comprising an ophthalmic microscope; a first and a second lens connected to each other by means of an arm and aligned along an axis X.

The first lens is placed in proximity to the ophthalmic microscope and the second lens is close to a patient.

The optical group further comprises a system for defogging a lens according to the present invention and of the type described above, placed in proximity to the second lens to hit the second lens with the pressurized fluid flow.

Further features and advantages of the present invention will become clearer from the indicative, and therefore non-limiting, description of a preferred but non-exclusive embodiment of a system for defogging a lens. The description will be set forth here below with reference to the appended drawings, provided solely for illustrative and thus non-limiting purposes, in which:
- figure 1 shows a schematic view of a fluid flow feeding system for defogging a lens, in accordance with the present invention, associated with the pan-fundoscopic system;
- figure 2 shows an enlargement of the system of figure 1;
- figure 2A shows an enlargement of a detail of figure 2;
- figure 3 shows an enlargement of a first constructive detail of the system of figure 1 in accordance with a first implementation solution;
- figures 4 and 5 show an enlargement of a constructive detail of the system of figure 1 in accordance with a second embodiment solution.

With reference to the aforementioned figures, a system for defogging a lens has been indicated overall with reference numeral 1.

Preferably the fluid flow is air which is pressurized by the system 1 to defog the lens.

The system 1 comprises a generation device 10 for continuously pressurizing the fluid flow fls_pres.

In particular, the generation device 10 has a hollow box-like structure, preferably rectangular, inside which a volume 11 is defined.

The box-like structure comprises at least a first 17 and a second 18 opening. The first opening 17 is arranged to be crossed by a channel 19 that conveys a fluid flow Fls inside the box-like structure, while the second opening 18 is arranged to convey the pressurized fluid flow Fls_pres towards the outside of the box-like structure.

Preferably, the box-like structure comprises a switch 15 configured to control the operation of the system 1.

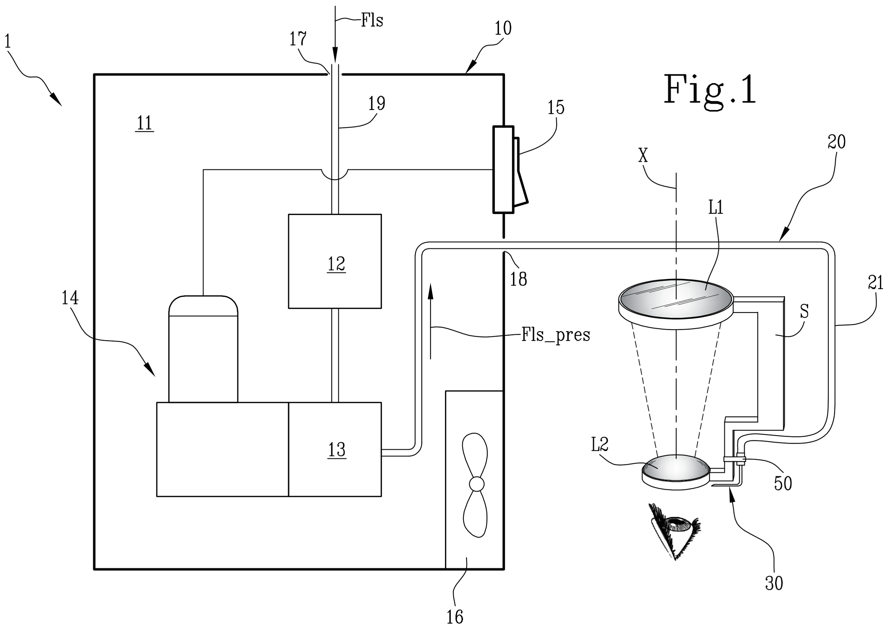

Referring to figure 1, it is noted that the volume 11 of the generation device 10 comprises at least a first 12 and a second 13 filtration device for filtering the fluid flow Fls in input to the generation device 10 and the fluid flow filtered by the first filtration device 12, respectively.

In particular, the first filtration device 12 is connected to a first end of the channel 19 by means of which the fluid flow Fls reaches the first filtration device 12.

In a non-limiting embodiment, not shown, the first filtration device 12 has a cylindrical structure comprising therein a filtration structure arranged to filter the fluid flow Fls and thus obtain a partially filtered fluid flow comprising a lower concentration of particulate than the fluid flow Fls inserted in input to the channel 19.

Referring again to figure 1, it is evident that the second filtration device 13 is connected downstream of the first filtration device 12.

Advantageously, the second filtration device 13 further filters the fluid flow in such a way as to further reduce the concentration of the particulate present in the fluid flow Fls in input to the channel 19.

Therefore, the first 12 and the second 13 filtration device determine the realization of a pressurized fluid flow Fls_pres usable in sterile environments, for example in the operating room.

The volume 11 of the generation device 10 further comprises a pressurization pump 14 arranged to suck air and pressurize the fluid flow continuously.

Preferably the pressurization pump 14 is a membrane pump that controls the operation and activation of the system 1.

In fact, the pressurization pump 14 is connected both to the switch 15 and to the first 12 and second 13 filtration device and is arranged to activate the filtration performed by the first filtration device 12 and the further filtration performed by the second filtration device 13, following an electrical signal received from the switch 15.

The switch 15 is arranged to transmit an electrical signal to the pressurization pump 14, following a pressure exerted on the switch itself, and to control the activation of the pressurization pump 14, of the first 12 and second 13 filtration device.

Typically, when the switch 15 transmits the electrical signal, the pressurization pump 14 is activated, which pressurizes the fluid flow filtered by the first filtration device 12.

Then, the second filtration device 13 receives the pressurized fluid flow Fls_pres from the mechanical pump 14 and further filters the fluid flow. Thereby the system 1 pressurizes and filters the fluid flow to defog the lens.

In other words, by performing an adequate filtration the fluid flow generated can also be used in the hospital environment.

As a result of the continuous and prolonged operation of the system 1, an excessive amount of heat may be produced within the generation device 10.

To solve this problem, the volume 11 of the generation device 10 further comprises a cooling fan 16 arranged to cool the volume 11 itself.

The generation device 10 further comprises a potentiometer for adjusting the speed of the flow of the generated air. In this way the flow rate of the fluid is controlled based on the amount of small drops adhered on the lens. In other words, if the amount of small drops on the lens is high, the potentiometer is controlled in such a way that the amount of air is greater and able to eliminate the drops by drying the lens.

The system 1 further comprises a transport device 20 connected to the generation device 10 and arranged to transport the pressurized fluid flow Fls_pres outside the generation device 10.

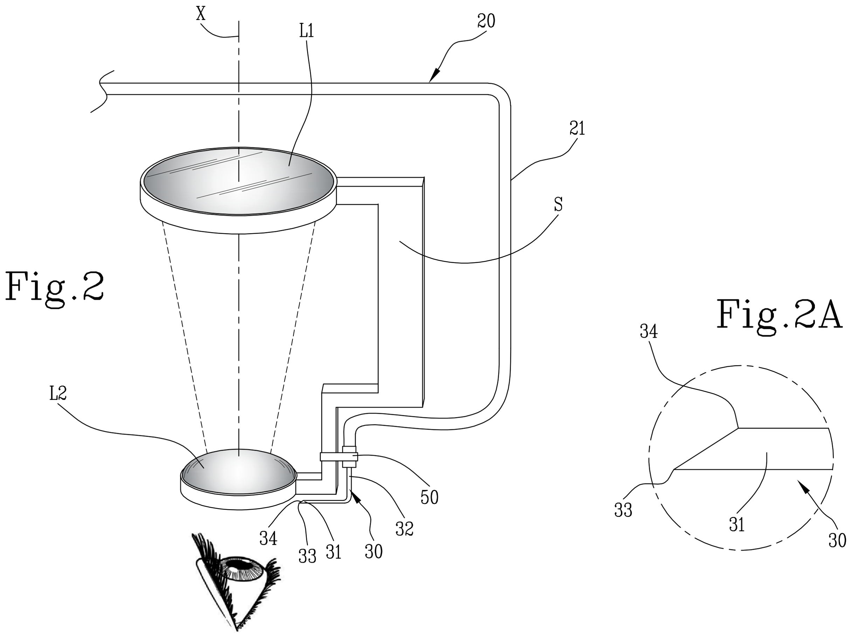

With reference to figures 1 and 2, it is evident that the transport device 20 comprises a conduit 21 connected to the second filtration device 13. This conduit 21 transports externally to the second filtration device 13 the pressurized fluid flow Fls_pres.

Preferably the conduit 21 of the transport device 20 is made of disposable silicone material. For example, the transport device 20 is a millipore filter.

Advantageously, the transport device 20 is flexible and adaptable. In this way, the transport device 20 can be used in different environments and conditions.

Preferably, the transport device 20 develops longitudinally in such a way as to reach extended lengths and, therefore, the generation system 10 can also be positioned in an environment external to a sterile environment in which the pressurized fluid flow Fls_pres is conveyed.

The system 1 further comprises at least one fluid flow fls_pres dispensing element 30 connected to the transport device 20 and arranged to dispense the pressurized fluid flow fls_pres. Preferably the pressurized fluid flow Fls_pres is dispensed in a laminar manner.

With reference to figures 1 and 2, it is evident that the dispensing element 30 transports externally to the transport device 20 the pressurized fluid flow Fls_pres.

In a non-limiting embodiment, not shown, the dispensing element 30 is connected to the transport device 20 by means of a junction element (e.g., a fitting) that minimizes the loss of pressurized fluid flow Fls_pres and isolates the pressurized fluid flow Fls_pres.

Referring to figure 2-2A, it is noted that the dispensing element 30 comprises a micro-conveyor 32. The latter has a typically "L" shape and is preferably made of metallic material.

Advantageously the dispensing of the pressurized fluid flow fls_pres is controlled more precisely.

In other words, the metallic structure of the dispensing element 30 allows greater control of the orientation and positioning thereof with respect to the lens L of interest.

On the end of the micro-conveyor 32 there is positioned a nozzle 31 arranged to dispense the fluid flow fls_pres tangentially to the lens L.

Advantageously, the pressurized fluid flow Fls_pres tangentially engages the surface of the lens L and, in the case where the lens L is substantially flat, allows to substantially preserve the laminar fluid flow.

Thus, there is no passage from a laminar to turbulent fluid flow.

In this way, when the system 1 is used in ophthalmic surgery, in particular in proximity to a patient's eye, it is avoided generating fluid flow vortices that may adversely influence the physiological balance and health of the patient.

In an exemplary and non-limiting embodiment shown in figure 2, the micro-conveyor 32 is connected to the transport device 20 by means of the end opposite to that of the nozzle 31.

Preferably, the micro-conveyor 32 has a diametrical size smaller than the diametrical size of the transport device 20.

Referring to figure 2A, it is noted that the nozzle 31 has a flute beak structure.

Preferably, when the system 1 is associated with the lens L, the most extended portion 33 of the nozzle 31 is in a distal position with respect to the lens and the least extended portion 34 of the nozzle 31 is in a proximal position with respect to the lens L.

Preferably the nozzle 31 is arranged to dispense the pressurized fluid flow Fls_pres tangentially and uniformly to the lens L.

In this way, the lens is defogged uniformly and homogeneously at every point.

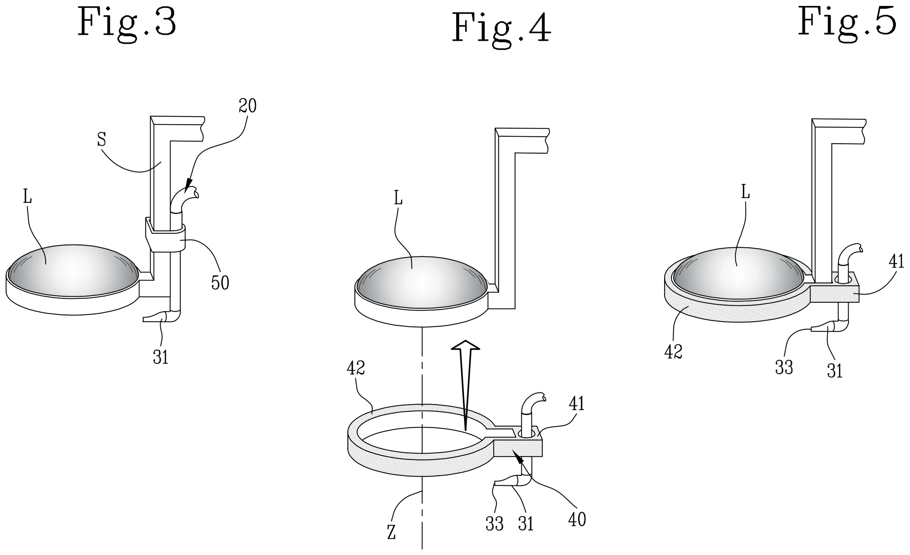

In an embodiment shown in figures 4 and 5, it is noted that the system 1 further comprises a support 40 arranged to position the dispensing element 30 and connect it to a lens L.

In particular, the support 40 comprises a hollow cylindrical structure 42 and a support element 41 connected outside the hollow cylindrical structure 42. The support element 41 has a hole defining an area occupied by the dispensing element 30.

In particular, the micro-conveyor 32 passes through the hole of the support element 41 and the nozzle 31 is placed at a pre-defined distance from the support element 41.

Advantageously, when the system 1 is associated with a lens L, the distance of the nozzle 31 from the lens is controlled more precisely and accurately.

Referring always to figures 4 and 5, it is evident that the hole present on the support element 41 is made at a distance from the edge equal to at least the length of the most extended portion 33 of the nozzle 31.

In other words, the end of the nozzle 31, preferably of the most extended portion 33, is distant from the longitudinal axis Z of the hollow cylindrical structure 42 by at least one size equal to the radius of the inner cylinder of the hollow cylindrical structure 42.

The present invention, described above, is associated with an optical group for surgical procedures.

The optical group comprises an ophthalmic microscope and a first L1 and a second L2 lens.

The first L1 and second L2 lenses are connected to each other by means of an arm S that allows the lenses to be supported and aligned with each other along an axis X.

In particular, the arm S is made in such a way as to place the first L1 and the second L2 lens at a distance to visualize the peripheral and central retina and the vitreous chamber of an eye, when the two lenses are associated with the ophthalmic microscope.

In other words, at least one lens, for example the second lens L2, is placed in proximity to the patient's eye.

The ophthalmic microscope is placed in proximity to the first lens L1, and in particular the ophthalmic microscope is aligned with the first lens L1.

Generally, the system 1, and in particular the dispensing element 30, is associated with the second lens L2. In this way, by positioning the system 1 in proximity to the second lens L2, the pressurized fluid flow Fls_pres hits the second lens L2.

The optical group comprises connection elements 50 for connecting the system 1 to the arm S of the first L1 and second L2 lens.

In this way, the system is stable. In addition, it is also possible to control the distance of the nozzle 31 of the dispensing element 30 from the second lens L2 by fixing, by means of the connection elements 50, the dispensing element 30 to a certain position of the arm S.

Advantageously, by controlling the distance of the nozzle 31 from the second lens L2, the system 1 defogs the lens uniformly.

Furthermore, the optical group comprises the above-described support 40 for further positioning the dispensing element 30.

In particular, as illustrated in figure 5, the support 40 has a counter-shape to the second lens L2 to engage therewith and further connect the dispensing element 30 to the second lens L2.

In this way the system 1 is fixed to the arm S with greater stability.

Advantageously, the present invention solves the drawbacks mentioned in the prior art and achieves the intended purposes.

In particular, the system 1 is able to defog a lens by dispensing a pressurized fluid flow Fls_pres, distributing the fluid flow in a homogeneous and uniform way, in such a way as to keep the visual field of the operator always sharp.

Even more advantageously, the system 1 allows to keep the visual field of the surgeon unchanged during the entire execution of a surgical operation, regardless of the time necessary to conclude the operation itself.

Furthermore, the system 1 has the advantage of allowing a lens to be positioned and aligned only once and more accurately with the patient's eye during a surgical operation.

Another advantage of the invention is to minimize the need to interrupt the surgical operation, without requiring extended times to perform the operation and thus reducing the condition of operational discomfort.

Yet another advantage of the invention is to reduce the total operation times, increase the number of procedures for a given operating room and, consequently, reduce the costs of a single surgical operation.

A further advantage of the invention is to have a system 1 capable of reducing the degradation of the lenses following sterilization cycles and of reducing the wear of the lenses following mechanical defogging by means of gauzes or fabrics.

## Claims

1. System (1) for defogging a lens (L), **characterised in that** it comprises:
a fluid flow (Fls) generation device (10) for pressurizing a continuous fluid flow (Fls_pres);
a transport device (20) connected to the generation device (10) and for transporting the fluid flow (Fls_pres) outside the generation device (10);
at least one fluid flow (Fls_pres) dispensing element (30) connected to the transport device (20) and having a nozzle (31) for dispensing the fluid flow (Fls_pres) in laminar form on the surface of the lens (L).

2. System according to the preceding claim, wherein the generation device (10) comprises:
a mechanical pump (14) for sucking air defining said fluid flow (Fls) and pressurizing the continuous fluid flow;
and at least one first filtration device (12) for filtering said fluid flow (Fls).

3. System according to the preceding claim, wherein said generation device (10) further comprises at least a second filtration device (13) arranged downstream of the first filtration device (12) for filtering the fluid flow (Fls) previously filtered by the first filtration device (12);

4. System according to the preceding claim, wherein said generation device (10) has a box-like structure defining a volume (11) for containing the pump (14), the first (12) and the second (13) filtration device; said generation device (10) further comprising a cooling fan (16) for cooling said volume (11).

5. System according to the preceding claim, wherein the box-like structure comprises a switch (15) connected to the mechanical pump (14) for controlling the activation of the first filtration device (12) and/or of the second filtration device (13) and/or of the mechanical pump (14).

6. System according to any one of the preceding claims, wherein the transport device (20) comprises a conduit (21) connected to the generation device (10) and to the dispensing element (30) for transporting externally to the generation device (10) the pressurized fluid flow (Fls_pres).

7. System according to the preceding claim wherein the conduit (21) comprises a millipore filter.

8. System according to any one of the preceding claims, wherein the dispensing element (30) comprises a micro conveyor (32) having a first end associated with the nozzle (31) for dispensing the fluid flow and a second end opposite to the first one and associated with the transport device (20).

9. System according to any one of the preceding claims, wherein the nozzle (31) has a bevelled structure for dispensing the pressurized fluid flow (Fls_pres) in laminar form and tangentially to the surface of the lens (L).

10. System according to any one of the preceding claims further comprising a support (40) for positioning the dispensing element (30) in proximity to the lens (L); wherein the support (40) comprises a hollow cylindrical structure (42) and a connection element (41) connected to each other; wherein the connection element (41) is engaged with the dispensing element (30) and wherein the hollow cylindrical structure (42) is engageable with the lens (L).

11. Optical group for surgical procedures comprising:
an ophthalmic microscope;
a first (L1) and a second (L2) lens connected to each other by means of an arm (S) and aligned along an axis X;
said first lens (L1) being placed in proximity to the ophthalmic microscope and said second lens (L2) being close to a patient;
**characterized in that** it comprises a system (1) according to one or more of the preceding claims,
placed in proximity to the second lens (L2) to hit said second lens (L2) with the pressurized fluid flow (Fls_pres).

12. Optical group according to the preceding claim, comprising connection elements (50) for connecting the system (1) to the arm (S) of the first (L1) and second (L2) lens.

13. Optical group according to claim 11, further comprising a support (40) according to claim 10.
